(19) Europäisches Patentamt
European Patent Office
Office européen des brevets

(11) Veröffentlichungsnummer: **0 545 167 A1**

# (12) EUROPÄISCHE PATENTANMELDUNG

(21) Anmeldenummer: **92119773.7**

(22) Anmeldetag: **20.11.92**

(51) Int. Cl.5: **C07D 207/452**, C07D 209/48, A01N 43/36, A01N 43/38

(30) Priorität: **02.12.91 DE 4139636**

(43) Veröffentlichungstag der Anmeldung: **09.06.93 Patentblatt 93/23**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Schallner, Otto, Dr.**
**Noldeweg 22**
**W-4019 Monheim(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**
Erfinder: **Vosswinkel, Renate, Dr.**
**Sankt-Matenus-Eck 14a**
**W-5067 Kürten-Bechen(DE)**

## (54) N-(4-Hydroxyphenyl)-Maleinimide und N-(4-hydroxyphenyl)-Tetrahydrophthazimide und ihre Verwendung als Herbizide.

(57) Die Erfindung betrifft neue N-Aryl-Stickstoffheterocyclen der allgemeinen Formel (I)

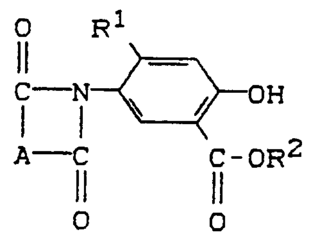

(I)

in welcher

$R^1$ für Wasserstoff oder Halogen steht,

$R^2$ für Wasserstoff oder für einen jeweils gegebenenfalls substituierten, geradkettigen oder verzweigten Rest der Reihe Alkyl, Alkenyl oder Cycloalkyl steht und

A für einen der Reste

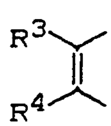

oder

EP 0 545 167 A1

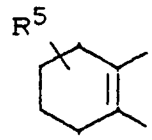

steht,
wobei
$R^3$ für Alkyl steht,
$R^4$ für Alkyl steht und
$R^5$ für Wasserstoff oder Alkyl steht,
mehrere Verfahren zu ihrer Herstellung und ihre Verwendung als Herbizde.

Die Erfindung betrifft neue N-Aryl-Stickstoffheterocyclen, mehrere Verfahren zu ihrer Herstellung und ihre Verwendung als Herbizide.

Es ist bekannt, daß bestimmte N-Aryl-Stickstoffheterocyclen wie beispielsweise die Verbindung 4-Chlor-1-(4-cyano-2-fluor-5-methoxycarbonylmethylthio-phenyl)-3,5-dimethyl-pyrazol herbizide Eigenschaften besitzen (vgl. z.B. DE 38 39 480). Weitere N-Aryl-Stickstoffheterocyclen mit herbiziden Wirkeigenschaften werden aber auch in der DE 38 35 168 und DE 38 19 439 beschrieben.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue N-Aryl-Stickstoffheterocyclen der allgemeinen Formel (I),

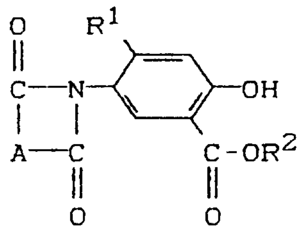

(I)

in welcher

$R^1$ für Wasserstoff oder Halogen steht,

$R^2$ für Wasserstoff oder für einen jeweils gegebenenfalls substituierten, geradkettigen oder verzweigten Rest der Reihe Alkyl, Alkenyl oder Cycloalkyl steht und

A für einen der Reste

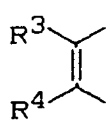

oder

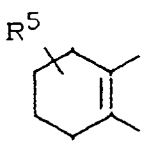

steht,

wobei

$R^3$ für Alkyl steht,

$R^4$ für Alkyl steht und

$R^5$ für Wasserstoff oder Alkyl steht,

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen N-Aryl-Stickstoffheterocyclen der allgemeinen Formel (I),

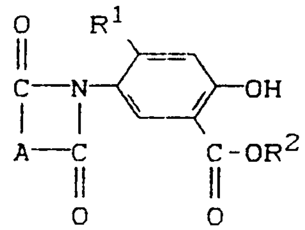

(I)

in welcher

$R^1$ für Wasserstoff oder Halogen steht,

$R^2$ für Wasserstoff oder für einen jeweils gegebenenfalls substituierten, geradkettigen oder verzweigten Rest der Reihe Alkyl, Alkenyl oder Cycloalkyl steht und

A für einen der Reste

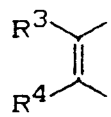

oder

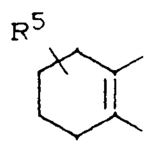

steht,

wobei

$R^3$ für Alkyl steht,

$R^4$ für Alkyl steht und

$R^5$ für Wasserstoff oder Alkyl steht,

erhält, wenn man

a) Anhydride der Formel (II)

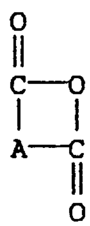

(II)

in welcher

A die oben angegebene Bedeutung hat,

mit Anilinderivaten der Formel (III),

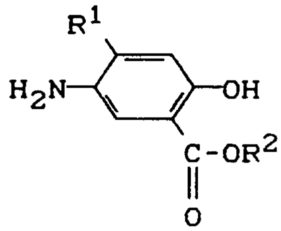

(III)

in welcher

$R^1$ und $R^2$ die oben angegebene Bedeutung haben,

gegebenenfalls in Gegenwart eines Verdünnungsmittels umsetzt und gegebenenfalls anschließend

b) die so erhältlichen N-Aryl-Stickstoffheterocyclen der Formel (Ia),

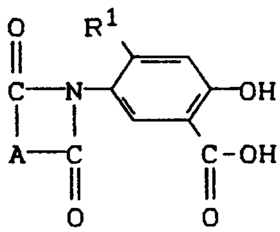

(Ia)

in welcher

$R^1$ und A die oben angegebene Bedeutung haben,

mit Alkoholen der Formel (V),

$R^{2-1}$-OH (IV)

in welcher

$R^{2-1}$ für einen jeweils gegebenenfalls substituierten Rest der Reihe Alkyl, Alkenyl oder Cycloalkyl steht,

gegebenenfalls in Gegenwart eines Verdünnungsmittels und in Gegenwart eines Reaktionshilfsmittels verestert.

Schließlich wurde gefunden, daß die neuen N-Aryl-Stickstoffheterocyclen der allgemeinen Formel (I) herbizide Eigenschaften besitzen.

Überraschenderweise zeigen die erfindungsgemäßen N-Aryl-Stickstoffheterocyclender allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen als die aus dem Stand der Technik bekannten N-Aryl-Stickstoffheterocyclen, wie beispielsweise die Verbindung 4-Chlor-1-(4-cyano-2-fluor-5-methoxycarbonylmethylthiophenyl)-3,5-dimethyl-pyrazol, welche chemisch und wirkungsmäßig naheliegende Verbindungen sind.

Die erfindungsgemäßen N-Aryl-Stickstoffheterocyclen sind durch die Formel (I) allgemein definiert. Bevorzugt sind Verbindungen der Formel (I), bei welchen

$R^1$ für Wasserstoff, Fluor, Chlor, Brom oder Iod steht,

$R^2$ für Wasserstoff oder für einen geradkettigen oder verzweigten Rest der Reihe Alkyl mit 1 bis 12 Kohlenstoffatomen, Halogenalkyl mit 1 bis 6 Kohlenstoffatomen und 1 bis 13 gleichen oder verschiedenen Halogenatomen, Alkoxyalkyl, Alkoxyalkoxyalkyl oder Alkoxycarbonylalkyl mit jeweils 1 bis 6 Kohlenstoffatomen in den einzelnen Alkylteilen, Alkenyl mit 2 bis 6 Kohlenstoffatomen oder für gegebenenfalls einfach oder mehrfach gleich oder verschieden durch geradkettiges oder verzweigtes Alkyl mit 1 bis 4 Kohlenstoffatomen substituiertes Cycloalkyl mit 3 bis 8 Kohlenstoffatomen steht und

A für einen der Reste

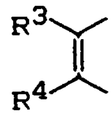

oder

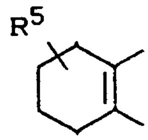

steht,
wobei

$R^3$ für geradkettiges oder verzweigtes Alkyl mit 1 bis 6 Kohlenstoffatomen steht,

$R^4$ für geradkettiges oder versweigtes Alkyl mit 1 bis 6 Kohlenstoffatomen steht und

$R^5$ für Wasserstoff oder für geradkettiges oder verzweigtes Alkyl mit 1 bis 6 Kohlenstoffatomen steht.

Besonders bevorzugt sind Verbindungen der Formel (I), bei welchen

$R^1$ für Wasserstoff, Fluor, Chlor oder Brom steht,

$R^2$ für Wasserstoff oder für einen geradkettigen oder verzweigten Rest der Reihe Alkyl mit 1 bis 8 Kohlenstoffatomen, Halogenalkyl mit 1 bis 4 Kohlenstoffatomen und 1 bis 9 gleichen oder verschiedenen Halogenatomen, Alkoxyalkyl, Alkoxyalkoxyalkyl oder Alkoxycarbonylalkyl mit jeweils 1 bis 4 Kohlenstoffatomen in den einzelnen Alkylteilen, Alkenyl mit 2 bis 4 Kohlenstoffatomen oder für gegebenenfalls einfach bis dreifach, gleich oder verschieden durch geradkettiges oder verzweigtes Alkyl mit 1 bis 3 Kohlenstoffatomen substituiertes Cycloalkyl mit 3 bis 7 Kohlenstoffatomen steht und

A für einen der Reste

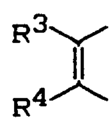

oder

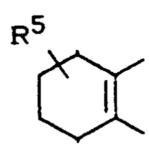

steht,
wobei

$R^3$ für geradkettiges oder verzweigtes Alkyl mit 1 bis 4 Kohlenstoffatomen steht,

$R^4$ für geradkettiges oder verzweigtes Alkyl mit 1 bis 4 Kohlenstoffatomen steht und

$R^5$ für Wasserstoff oder für geradkettiges oder verzweigtes Alkyl mit 1 bis 4 Kohlenstoffatomen steht.

Ganz besonders bevorzugt sind Verbindungen der Formel (I), bei welchen

$R^1$ für Wasserstoff oder Fluor steht,

$R^2$ für Wasserstoff oder für einen jeweils gegebenenfalls geradkettigen oder verzweigten Rest der Reihe Alkyl mit 1 bis 6 Kohlenstoffatomen, Halogenalkyl mit 1 bis 3 Kohlenstoffatomen und 1 bis 7 gleichen oder verschiedenen Halogenatomen, Alkoxyalkyl, Alkoxyalkoxyalkyl oder Alkoxycarbonylalkyl mit jeweils 1 bis 3 Kohlenstoffatomen in den einzelnen Alkylteilen, Alkenyl mit 2 bis 3 Kohlenstoffatomen oder für jeweils gegebenenfalls einfach oder zweifach durch Methyl substituiertes Cyclopropyl, Cyclopentyl oder Cyclohexyl steht,

A für einen der Reste

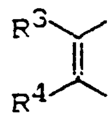

oder

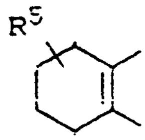

steht,

wobei

$R^3$ für Methyl steht,

$R^4$ für Methyl steht und

$R^5$ für Wasserstoff, Methyl oder Ethyl steht.

Verwendet man beispielsweise 3,4,5,6-Tetrahydrophthalsäureanhydrid und 3-Ethoxycarbonyl-4-hydroxy-anilin als Ausgangsstoffe, so läßt sich der Reaktionsablauf des erfindungsgemäßen Verfahrens (a) durch das folgende Formelschema darstellen:

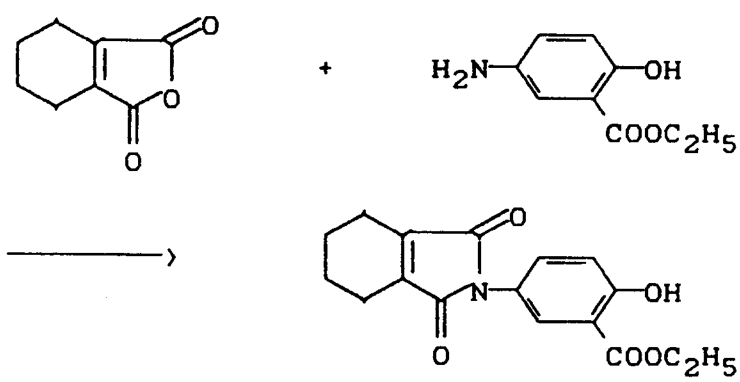

Verwendet man beispielsweise 2-Hydroxy-5-(2,5-dioxo-3,4-dimethyl-3-pyrrolin-1-yl)-benzoesäure und Ethoxyethanol als Ausgangsstoffe sowie Oxalylchlorid als Reaktionshilfsmittel, so läßt sich der Reaktionsablauf des erfindungsgemäßen Verfahrens (b) durch das folgende Formelschema darstellen:

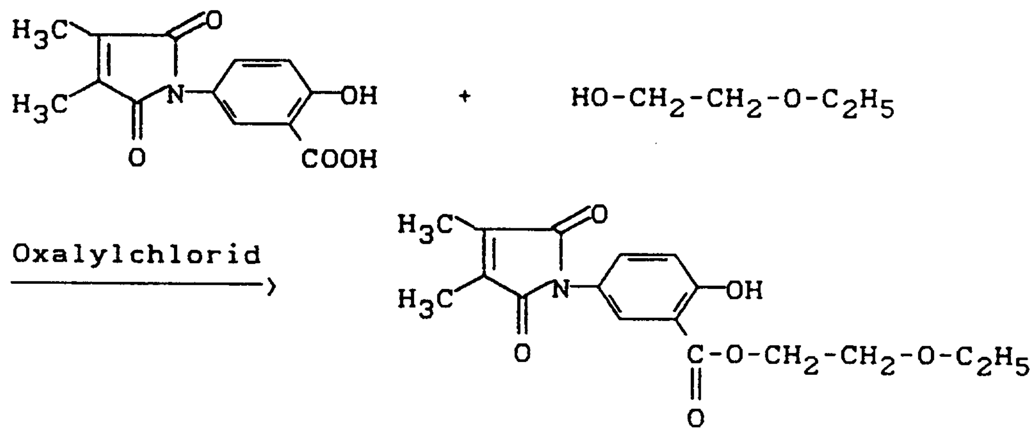


Die zur Durchführung des erfindungsgemäßen Verfahrens (a) als Ausgangsstoffe benötigten Anhydride sind durch die Formel (II) allgemein definiert. In dieser Formel (II) steht A vorzugsweise für diejenigen Reste, die bereits im Zusammenhang mit der Beschreibung der erfindungsgemäßen Verbindungen der Formel (I) als bevorzugt für diesen Substituenten genannt wurden.

Die Anhydride der Formel (II) sind allgemein bekannt (vgl. z.B. Gazz. Chim. Ital. 57, 300-311 [1927]).

Die zur Durchführung des erfindungsgemäßen Verfahrens (a) weiterhin als Ausgangsstoffe benötigten Anilin-Derivate sind durch die Formel (III) allgemein definiert.

In dieser Formel (111) stehen $R^1$ und $R^2$ vorzugsweise für diejenigen Reste, die bereits im Zusammenhang mit der Beschreibung der erfindungsgemäßen Verbindungen der Formel (I) als bevorzugt für diese Substituenten genannt wurden.

Die Anilin-Derivate der Formel (III) sind ebenfalls allgemein bekannt (vgl. z.B. EP 114 734; US 4.036.951; Collect. Czech. Commun. 29, 730 [1964]).

Die zur Durchführung des erfindungsgemäßen Verfahrens (b) als Edukte erforderlichen N-Aryl-Stickstoffheterocyclen sind durch die Formel (Ia) allgemein definiert. In dieser Formel (Ia) stehen $R^1$ und A vorzugsweise für diejenigen Reste, die bereits im Zusammenhang mit der Beschreibung der erfindungsgemäßen Stoffe der Formel (I) als bevorzugt für diese Substituenten genannt wurden.

Die N-Aryl-Stickstoffheterocyclen der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Edukte erforderlichen Alkohole sind durch die Formel (IV) allgemein definiert. In dieser Formel (IV) steht $R^{2-1}$ vorzugsweise für diejenigen Reste, die bereits im Zusammenhang mit der Beschreibung der erfindungsgemäßen Stoffe der Formel (I) als bevorzugt für den Substituenten $R^2$ genannt wurden, mit Ausnahme des Wasserstoffrestes.

Die Alkohole der Formel (IV) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren, wie beispielsweise Essigsäure oder p-Toluolsulfonsäure, Anhydride wie Acetanhydrid oder Säurechloride wie Acetylchlorid als Reaktionshilfsmittel. Es ist auch möglich, andere übliche wasserabspaltende Mittel, wie beispielsweise N,N'-Dicyclohexylcarbodiimid als Reaktionshilfsmittel zu verwenden. Außerdem ist es auch möglich, die als Reaktionshilfsmittel verwendeten Säuren in entsprechendem Überschuß gleichzeitig als Verdünnungsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Das erfindungsgemäße Verfahren (a) wird üblicherweise unter Normaldruck durchgeführt.
Es ist jedoch auch möglich, unter erhöhtem oder vermindertem Druck zu arbeiten.

Zur Durchführung des erfindungsgemäßen Verfahrens (a) setzt man pro Mol Anhydrid der Formel (II) im allgemeinen 0,8 bis 1,5 Mol, vorzugsweise 1,0 bis 1,2 Mol Anilin-Derivat der Formel (III) und gegebenenfalls 0,5 bis 15,0 Mol Reaktionshilfsmittel ein. Die Reaktionsdurchführung, Aufarbeitung und Isolierung der Reaktionsprodukte erfolgt nach bekannten Verfahren (vgl. hierzu beispielsweise DE 37 12 987 oder die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Sulfoxide, wie Dimethylsulfoxid.

Das erfindungsgemäße Verfahren (b) kann gegebenenfalls in Gegenwart eines geeigneten Reaktionshilfsmittels durchgeführt werden. Als solche kommen übliche Säurehalogenidbildner wie Thionylchlorid, Sulfurylchlorid, Phosphortrichlorid, Phosphorpentachlorid, Phosphoroxychlorid oder Oxalylchlorid infrage.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 80 °C, vorzugsweise bei Temperaturen zwischen 20 °C und 50 °C.

Zur Durchführung des erfindungsgemäßen Verfahrens (b) setzt man pro Mol N-Aryl-Stickstoffheterocyclus der Formel (Ia) im allgemeinen 1,0 bis 10,0 Mol, vorzugsweise 1,0 bis 5,0 Mol Alkohol der Formel (IV) und gegebenenfalls 1,0 bis 3,0 Mol, vorzugsweise 0,5 bis 2,0 Mol Reaktionshilfsmittel ein.
Die Reaktionsdurchführung, Aufarbeitung und Isolierung der Reaktionsprodukte erfolgt nach bekannten Verfahren (vgl. auch die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirksfoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Weizen, Gerste,

Raps oder Soja einsetzen.

Daneben greifen die erfindungsgemäßen Wirkstoffe auch in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze, sowie von den auf die Pflanzen oder ihre Umgebung ausgebrachten Wirkstoffmengen und von der Art der Applikation. In jedem Fall sollen Wachstumsregulatoren die Kulturpflanzen in bestimmter, gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen, wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen`

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol, sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor,

Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und loxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1:

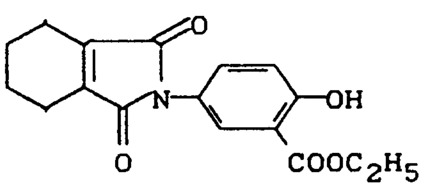

(Verfahren (a))

Zu einer Lösung von 7,3 g (0,04 Mol) 5-Amino-2-hydroxybenzoesäuremethylester (vgl. z.B. EP 114 734) in 100 ml Eisessig gibt man 6,4 g (0,042 Mol) 3,4,5,6-Tetrahydrophthalsäureanhydrid und rührt 16 Stunden bei 80 °C bis 85 °C. Zur Aufarbeitung wird die abgekühlte Reaktionsmischung im Vakuum eingeengt, der Rückstand in 150 ml Dichlormethan aufgenommen, mit gesättigter Natriumhydrogencarbonatlösung und Wasser gewaschen, über Natriumsulfat getrocknet und abermals im Vakuum eingeengt. Der Rückstand wird mit Petrolether verrührt, abfiltriert und getrocknet.

Man erhält 10,5 g (83% der Theorie) N-(3-Ethoxycarbonyl-4-hydroxyphenyl)-3,4,5,6-tetrahydrophthalimid mit Schmelzpunkt 90 °C.

Beispiel 2:

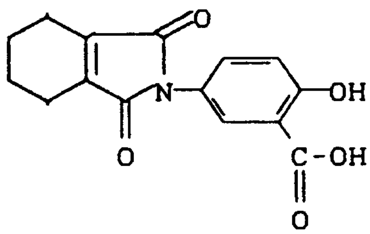

(Verfahren (a))

Zu 24,6 g (0,15 Mol; 93%ig) 5-Amino-2-hydroxy-benzoesäure in 240 ml Eisessig gibt man 22,8 g (0,15 Mol) 3,4,5,6-Tetrahydrophthalsäureanhydrid und rührt 12 bis 16 Stunden bei Rückflußtemperatur. Zur Aufarbeitung wird die abgekühlte Reaktionsmischung im Vakuum eingeengt, der Rückstand mit 360 ml einer Mischung aus Petrolether und Essigester (30:1) verrührt, das kristalline Produkt abgesaugt und getrocknet.

Man erhält 41 g (95% der Theorie) N-(3-Hydroxycarbonyl-4-hydroxyphenyl)-3,4,5,6-tetrahydrophthalimid mit Schmelzpunkt 240 °C.

Beispiel 3:

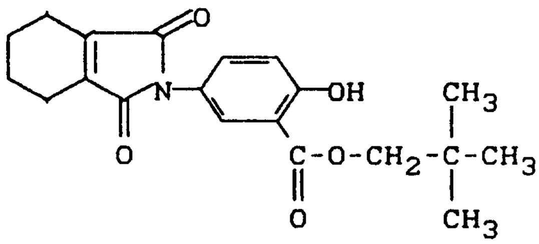

(Verfahren (b))

Zu 2,9 g (0,01 Mol) N-(5-Hydroxycarbonyl-4-hydroxyphenyl)-3,4,5,6-tetrahydrophthalimid in 50 ml trockenem Dichlormethan gibt man 1,4 g (0,011 Mol) Oxalylchlorid und rührt bei Raumtemperatur bis zur Beendigung der Gasentwicklung (ca. 30 Stunden). Die entstandene Lösung wird im Vakuum vom Lösungsmittel befreit, der Rückstand in 80 ml Tetrachlorkohlenstoff suspendiert und mit 0,9 g (0,01 Mol, 98%ig) 2,2-Dimethyl-1-propanol versetzt und für 16 Stunden auf Rückflußtemperatur erhitzt. Zur Aufarbeitung wird die abgekühlte Reaktionsmischung nacheinander mit Wasser und gesättigter Natriumhydrogencarbonatlösung gewaschen, über Magnesiumsulfat getrocknet und abermals im Vakuum eingeengt.

Man erhält 3,2 g (90% der Theorie) N-[3-(2,2-Dimethyl-1-propyloxycarbonyl)-4-hydroxyphenyl]-3,4,5,6-tetrahydrophthalimid als Öl.

$^1$H-NMR ($CDCl_3$/Tetrasilylmethan):

$\delta$ = 1,03 (s, 9H); 1,83 (m, 4H); 2,43 (m, 4H); 4,05 (s, 2H); 7,06 (d, 1H); 7,40 (m, 1H), 7,85 (d, 1H) ppm

In entsprechender Weise und gemäß den allgemeinen Angaben zur Herstellung erhält man die folgenden N-Aryl-Stickstoffheterocyclen der allgemeinen Formel (I);

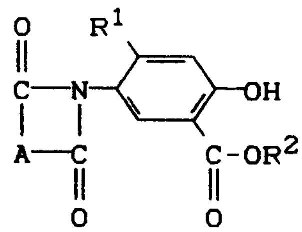 (I)

| Bsp.-Nr. | 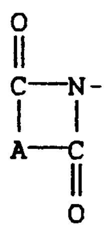 | $R^1$ | $R^2$ | physikalische Eigenschaften |
|---|---|---|---|---|
| 4 | 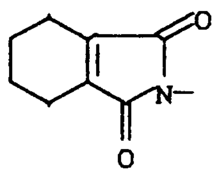 | H | $-CH(CH_3)_2$ | $^1H$-NMR*): 7,05 (d, 1H) |
| 5 | 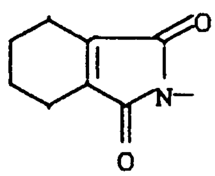 | H | $-CH_2-CH_2-CH_3$ | Fp.: 75° C |
| 6 | 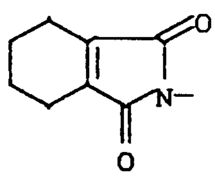 | H | $-CH_2-CH(CH_3)_2$ | Fp.: 67° C |
| 7 | 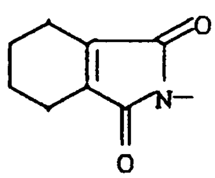 | H | $-CH(CH_3)-CH_2$<br>\|<br>$CH_3$ | $^1H$-NMR*): 7,05 (d, 1H) |

| Bsp.-Nr. | 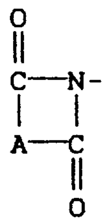 | $R^1$ | $R^2$ | physikalische Eigenschaften |
|---|---|---|---|---|
| 8 | 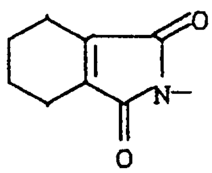 | H | $-C(CH_3)_3$ | Fp.: $108^{\circ}$ C |
| 9 | 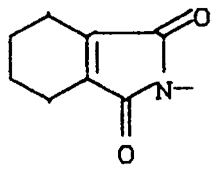 | H | 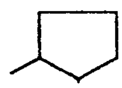 | Fp.: $102^{\circ}$ C |
| 10 | 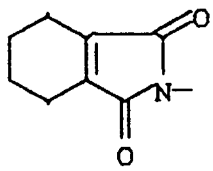 | H | $CH_3$ | Fp.: $137^{\circ}$ C |
| 11 | 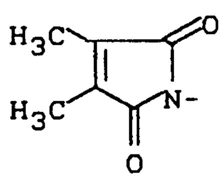 | H | $CH_3$ | Fp.: $148^{\circ}$ C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichssubstanz eingesetzt:

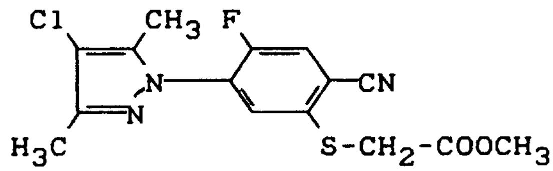

(A)

(vgl. z.B. DE 38 39 480)

Beispiel A:

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß Herstellungsbeispiel 1.

Beispiel B:

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß Herstellungsbeispiel 1.

**Patentansprüche**

**1.** N-Aryl-Stickstoffheterocyclen der allgemeinen Formel (I),

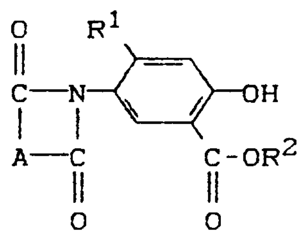

(I)

dadurch gekennzeichnet, daß

- $R^1$ für Wasserstoff oder Halogen steht,
- $R^2$ für Wasserstoff oder für einen jeweils gegebenenfalls substituierten, geradkettigen oder verzweigten Rest der Reihe Alkyl, Alkenyl oder Cycloalkyl steht und
- A für einen der Reste

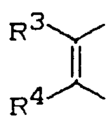

oder

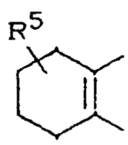

steht,
wobei

- $R^3$ für Alkyl steht,
- $R^4$ für Alkyl steht und
- $R^5$ für Wasserstoff oder Alkyl steht.

**2.** N-Aryl-Stickstoffheterocyclen der allgemeinen Formel (I) gemäß Anspruch 1, dadurch gekennzeichnet, daß

- $R^1$ für Wasserstoff, Fluor, Chlor, Brom oder Iod steht,
- $R^2$ für Wasserstoff oder für einen geradkettigen oder verzweigten Rest der Reihe Alkyl mit 1 bis 12 Kohlenstoffatomen, Halogenalkyl mit 1 bis 6 Kohlenstoffatomen und 1 bis 13 gleichen oder verschiedenen Halogenatomen, Alkoxyalkyl, Alkoxyalkoxyalkyl oder Alkoxycarbonylalkyl mit jeweils 1 bis 6 Kohlenstoffatomen in den einzelnen Alkylteilen, Alkenyl mit 2 bis 6 Kohlenstoffatomen oder für gegebenenfalls einfach oder mehrfach gleich oder verschieden durch geradkettiges oder verzweigtes Alkyl mit 1 bis 4 Kohlenstoffatomen substituiertes Cycloalkyl mit 3 bis 8 Kohlenstoffatomen steht und
- A für einen der Reste

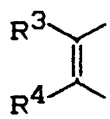

oder

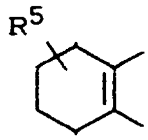

steht,
wobei

$R^3$ für geradkettiges oder verzweigtes Alkyl mit 1 bis 6 Kohlenstoffatomen steht,
$R^4$ für geradkettiges oder verzweigtes Alkyl mit 1 bis 6 Kohlenstoffatomen steht und
$R^5$ für Wasserstoff oder für geradkettiges oder verzweigtes Alkyl mit 1 bis 6 Kohlenstoffatomen steht.

**3.** N-Aryl-Stickstoffheterocyclen der allgemeinen Formel (I) gemäß Anspruch 1, dadurch gekennzeichnet, daß

$R^1$ für Wasserstoff, Fluor, Chlor oder Brom steht,
$R^2$ für Wasserstoff oder für einen geradkettigen oder verzweigten Rest der Reihe Alkyl mit 1 bis 8 Kohlenstoffatomen, Halogenalkyl mit 1 bis 4 Kohlenstoffatomen und 1 bis 9 gleichen oder verschiedenen Halogenatomen, Alkoxyalkyl, Alkoxyalkoxyalkyl oder Alkoxycarbonylalkyl mit jeweils 1 bis 4 Kohlenstoffatomen in den einzelnen Alkylteilen, Alkenyl mit 2 bis 4 Kohlenstoffatomen oder für gegebenenfalls einfach bis dreifach, gleich oder verschieden durch geradkettiges oder verzweigtes Alkyl mit 1 bis 3 Kohlenstoffatomen substituiertes Cycloalkyl mit 3 bis 7 Kohlenstoffatomen steht und
A für einen der Reste

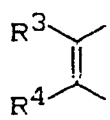

oder

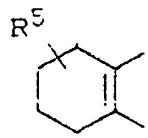

steht,
wobei

$R^3$ für geradkettiges oder verzweigtes Alkyl mit 1 bis 4 Kohlenstoffatomen steht,
$R^4$ für geradkettiges oder verzweigtes Alkyl mit 1 bis 4 Kohlenstoffatomen steht und
$R^5$ für Wasserstoff oder für geradkettiges oder verzweigtes Alkyl mit 1 bis 4 Kohlenstoffatomen steht.

**4.** N-Aryl-Stickstoffheterccyclen der allgemeinen Formel (I) gemäß Anspruch 1, dadurch gekennzeichnet, daß

$R^1$ für Wasserstoff oder Fluor steht,
$R^2$ für Wasserstoff oder für einen jeweils gegebenenfalls geradkettigen oder verzweigten Rest der Reihe Alkyl mit 1 bis 6 Kohlenstoffatomen, Halogenalkyl mit 1 bis 3 Kohlenstoffatomen und 1 bis 7 gleichen oder verschiedenen Halogenatomen, Alkoxyalkyl, Alkoxyalkoxyalkyl oder Alkoxycarbonylalkyl mit jeweils 1 bis 3 Kohlenstoffatomen in den einzelnen Alkylteilen, Alkenyl mit 2 bis 3 Kohlenstoffatomen oder für jeweils gegebenenfalls einfach oder zweifach durch Methyl substituiertes Cyclopropyl, Cyclopentyl oder Cyclohexyl steht,
A für einen der Reste

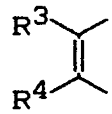

oder

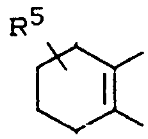

steht,
wobei
$R^3$ für Methyl steht,
$R^4$ für Methyl steht und
$R^5$ für Wasserstoff, Methyl oder Ethyl steht.

**5.** Verfahren zur Herstellung von N-Aryl-Stickstoffheterocyclen der allgemeinen Formel (I) gemäß Anspruch 1,

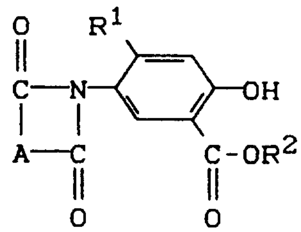

(I)

in welcher
$R^1$ für Wasserstoff oder Halogen steht,
$R^2$ für Wasserstoff oder für einen jeweils gegebenenfalls substituierten, geradkettigen oder verzweigten Rest der Reihe Alkyl, Alkenyl oder Cycloalkyl steht und
A für einen der Reste

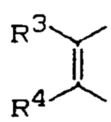

oder

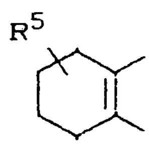

steht,
wobei
$R^3$ für Alkyl steht,

$R^4$ für Alkyl steht und
$R^5$ für Wasserstoff oder Alkyl steht,
dadurch gekennzeichnet, daß man
a) Anhydride der Formel (II)

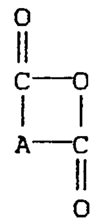

(II)

in welcher
A die oben angegebene Bedeutung hat,
mit Anilinderivaten der Formel (III),

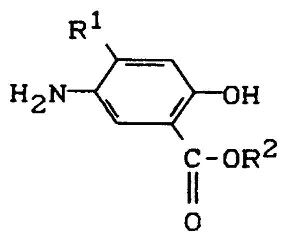

(III)

in welcher
$R^1$ und $R^2$ die oben angegebene Bedeutung haben,
gegebenenfalls in Gegenwart eines Verdünnungsmittels umsetzt und gegebenenfalls anschließend
b) die so erhältlichen N-Aryl-Stickstoffheterocyclen der Formel (Ia),

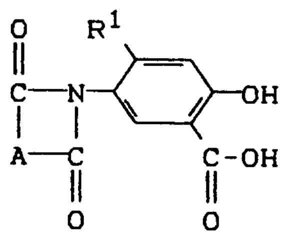

(Ia)

in welcher
$R^1$ und A die oben angegebene Bedeutung haben,
mit Alkoholen der Formel (V),

$R^{2-1}$-OH (IV)

in welcher
$R^{2-1}$ für einen jeweils gegebenenfalls substituierten Rest der Reihe Alkyl, Alkenyl oder Cycloalkyl steht,
gegebenenfalls in Gegenwart eines Verdünnungsmittels und in Gegenwart eines Reaktionshilfsmittels verestert.

**6.** Herbizide Mittel, gekennzeichnet durch einen Gehalt an mindestens einem N-Aryl-Stickstoffheterocyclus der Formel (I) gemäß den Ansprüchen 1 bis 5.

**7.** Verfahren zur Bekämpfung von unerwünschten Pflanzen, dadurch gekennzeichnet, daß man N-Aryl-Stickstoffheterocyclen der Formel (I) gemäß den Ansprüchen 1 bis 5 auf unerwünschte Pflanzen und/oder ihren Lebensraum einwirken läßt.

**8.** Verwendung von N-Aryl-Stickstoffheterocyclen der Formel (I) gemäß den Ansprüchen 1 bis 5 zur Bekämpfung von unerwünschten Pflanzen.

**9.** Verfahren zur Herstellung von herbiziden Mitteln, dadurch gekennzeichnet, daß man N-Aryl-Stickstoffheterocyclen der Formel (I) gemäß den Ansprüchen 1 bis 5 mit Streckmitteln und/oder oberflächenaktiven Substanzen vermischt.

**10.** N-Aryl-Stickstoffheterocyclen der allgemeinen Formel (Ia)

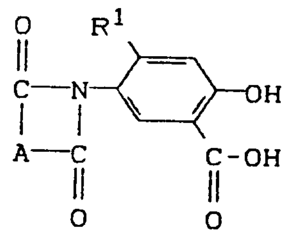

(Ia)

dadurch gekennzeichnet, daß

$R^1$ für Wasserstoff oder Halogen steht,

A für einen der Reste

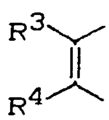

oder

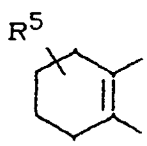

steht,

wobei

$R^3$ für Alkyl steht,

$R^4$ für Alkyl steht und

$R^5$ für Wasserstoff oder Alkyl steht.

**11.** N-Aryl-Stickstoffheterocyclen der allgemeinen Formel (Ia) gemäß Anspruch 10, dadurch gekennzeichnet, daß

$R^1$ für Wasserstoff, Fluor, Chlor, Brom oder Jod steht,

A für einen der Reste

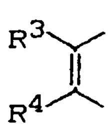

oder

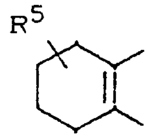

steht, wobei

$R^3$ für geradkettiges oder verzweigtes Alkyl mit 1 bis 6 Kohlenstoffatomen steht,

$R^4$ für geradkettiges oder verzweigtes Alkyl mit 1 bis 6 Kohlenstoffatomen steht und

$R^5$ für Wasserstoff oder für geradkettiges oder verzweigtes Alkyl mit 1 bis 6 Kohlenstoffatomen steht.

Europäisches Patentamt

# EUROPÄISCHER RECHERCHENBERICHT

Nummer der Anmeldung

EP 92 11 9773

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | DE-A-3 819 439 (BAYER)<br>14. Dezember 1989<br>* das ganze Dokument *<br>--- | 1-11 | C07D207/452<br>C07D209/48<br>A01N43/36<br>A01N43/38 |
| D,A | DE-A-3 839 480 (BAYER)<br>31. Mai 1990<br>* das ganze Dokument *<br>--- | 1-11 | |
| D,A | DE-A-3 835 168 (BAYER)<br>19. April 1990<br>* das ganze Dokument *<br>--- | 1-11 | |
| A | EP-A-0 338 987 (CIBA-GEIGY)<br>2. Oktober 1989<br>* das ganze Dokument *<br>--- | 1-11 | |
| Y | PATENT ABSTRACTS OF JAPAN<br>vol. 6, no. 85 (C-103)22. Mai 1982<br>& JP-P-57 016 804 ( IHARA CHEM. IND. ) 28. Januar 1982<br>* Zusammenfassung *<br>* N-(3-Carboxy-4-hydroxy-phenyl)-maleinimid, RN 65833-13-8 *<br>--- | 1-11 | RECHERCHIERTE SACHGEBIETE (Int. Cl.5 )<br>C07D |
| Y | GB-A-2 071 100 (NIPPON KAYAKU KK)<br>16. September 1981<br>* das ganze Dokument *<br>* 5-(1,3,4,5,6,7-hexahydro-1,3-dioxo-2H-isoindol-2-yl)-2-propoxy-benzoesäure, 1-methylethylester, RN 81663-26-5 *<br>----- | 1-11 | |

Der vorliegende Recherchenbericht wurde für alle Patentansprüche erstellt

| Recherchenort | Abschlußdatum der Recherche | Prüfer |
|---|---|---|
| DEN HAAG | 04 MAERZ 1993 | Bernd Kissler |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)